# EUROPEAN PATENT APPLICATION

(11) **EP 1 712 173 A2**
(43) Date of publication of application: **18.10.2006**
(21) Application number: 06290255.6
(22) Date of filing: 14.02.2006
(51) Int. Cl.: A47L 7/04, A47L 9/00, A47L 9/10

(54) **Vacuum cleaner**

(30) Priority: 11.04.2005 KR 2005029820
(71) Applicant: Samsung Gwangju Electronics Co., Ltd., Gwangju-city (KR)
(72) Inventor: Oh, Jang-keun, Seo-gu Gwangju-city (KR); Seo, Ji-won, Gwangsan-gu Gwangjiu-city (KR)
(74) Representative: Blot, Philippe Robert Emile

(57) **Abstract**

A vacuum cleaner (100) comprises a body with a dust-collecting chamber (151) and a motor driving chamber (155); a suction assembly (110) with an air suction path (113); a dust-collecting unit formed in the dust-collecting chamber (151) and collecting dusts or contaminants in an air; a motor assembly formed in the motor driving chamber and generating an air suction force; and an aerosol-generating unit (170) provided in an air path where the air drawn from the suction assembly flows, and spraying an antibiotic and sterilization material as a minute particle size.

## Description

### CROSS-REFERENCE TO RELATED APPLICATIONS

This application claims the benefit of Korean Patent Application No. 2005-29820 filed on April 11, 2005, in the Korean Intellectual Property Office, the disclosure of which is incorporated herein by reference in its entirety.

### BACKGROUND OF THE INVENTION

### 1. Field of the Invention

The present invention relates to a vacuum cleaner. More particularly, the present invention relates to a vacuum cleaner with sterilization and antibiotic functions against various bacteria and germs in dusts or contaminants drawn in the vacuum cleaner.

### 2. Description of the Related Art

Generally, a vacuum cleaner draws dusts or contaminants into a dust-collecting chamber by a suction force generated according to the driving of a motor in a driving chamber of a main body. The collected dusts or contaminants are kept or left in a dust bag or a dust cup for a long time so that various bacteria can be inhabited and propagated.

To solve the above problems, silver nano particles are additionally coated to the dust bag or parts (plastic injection molding material) around an air path, or parts are molded and manufactured by material with the silver nano particles. However, the silver nano particles are fixedly exposed only to the surface of parts so that the bacteria only on the surface can be sterilized, and the sterilization effect decreases.

### SUMMARY OF THE INVENTION

The present invention has been conceived to solve the above-mentioned problems occurring in the prior art, and an aspect of the present invention is to provide a vacuum cleaner having a silver nano particle generator which sprays a silver nano particle onto an air path so that an antibiotic and sterilization effect increases.

In order to achieve the above aspects, there is provided a vacuum cleaner comprising a body with a dust-collecting chamber and a motor driving chamber, a suction assembly with an air suction path, a dust collecting unit formed in the dust-collecting chamber and collecting dusts or contaminants in an air, a motor assembly formed in the motor driving chamber and generating an air suction force, and an aerosol-generating unit provided in an air path where the air drawn from the suction assembly flows, and spraying an antibiotic and sterilization material as a minute particle size.

The aerosol-generating unit may be formed in the air suction path. The aerosol-generating unit may be formed in the dust-collecting chamber.

The antibiotic and sterilization material may comprise an Argentum or silver (Ag) material.

The aerosol-generating unit may be periodically operated while the vacuum cleaner performs cleaning.

According to the second embodiment of the present invention, there is provided a vacuum cleaner comprising a body with a dust collecting chamber and a motor driving chamber, a suction assembly connected with the body and drawing in an air, an extension pipe assembly connecting the suction assembly and the dust collecting chamber of the body, a dust collecting unit formed in the dust-collecting chamber and collecting dusts or contaminants in the air, a motor assembly formed in the motor driving chamber and generating an air suction force, and an aerosol-generating unit formed at an inflow opening side of the body connected with the extension pipe assembly and spraying an antibiotic and sterilization material as a minute particle size.

According to the second embodiment of the present invention, there is provided a vacuum cleaner comprising a body with a motor-driving chamber and a dust-collecting chamber provided up and down therein, a suction assembly provided at a lower portion of the body and having an air suction path, a motor assembly provided in the motor-driving chamber, a dust-collecting unit provided in the dust-collecting chamber, and an aerosol-generating unit provided in the air suction path of the suction assembly and spraying an antibiotic and sterilization material as a fine particle.

If embodiments of the present invention are applied, the vacuum cleaner comprises a silver nano aerosol generator and sprays a silver nano aerosol material onto an air path with a drawn air moving therein such that the silver nano particle is activated and the sterilization effect can be maximized.

### BRIEF DESCRIPTION OF THE DRAWINGS

The above and other aspects, features and advantages of the present invention will be more apparent from the following detailed description taken with reference to the accompanying drawings, in which:

FIG. 1 is a sectional view of a canister type vacuum cleaner according to an embodiment of the present invention;

FIG. 2 is an enlarged-sectional view of a part of the canister type vacuum cleaner according to an embodiment of the present invention;

FIG. 3A is a sectional view of a small size of aerosol-generating unit applied to an embodiment of the present invention;

FIG. 3B is a sectional view of the aerosol-generating unit taken on III-III line of FIG 3A;

FIG. 4 is a view of an upright type vacuum cleaner according to another embodiment of the present invention;

FIG. 5 is a bottom view of a suction assembly of FIG. 4; and

FIG. 6 is an enlarged view of V portion of FIG. 5.

### DETAILED DESCRIPTION OF THE EXEMPLARY EMBODIMENTS

Exemplary embodiments of the present invention will be described in detail with reference to the annexed drawings. In the drawings, the same elements are denoted by the same reference numerals throughout the drawings. In the following description, detailed descriptions of known functions and configurations incorporated herein have been omitted for conciseness and clarity.

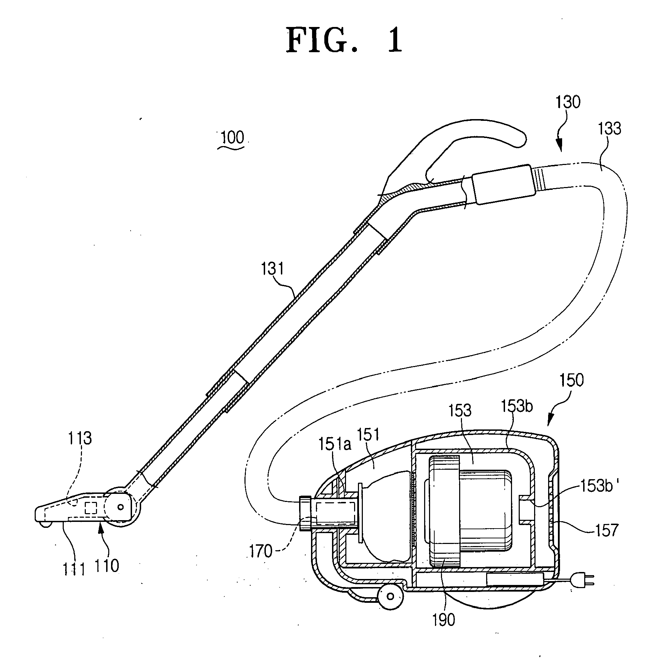

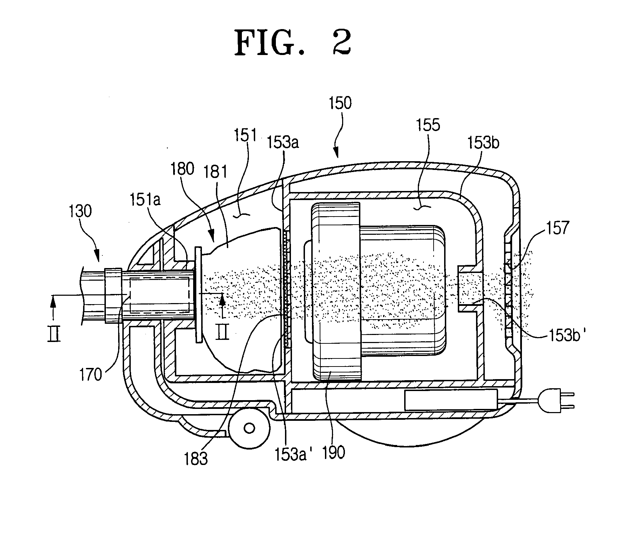

FIG. 1 is a sectional view of a canister type vacuum cleaner according to an embodiment of the present invention, and FIG. 2 is an enlarged view of a part of a canister type vacuum cleaner according to an embodiment of the present invention.

Referring to FIG 1, a vacuum cleaner 100 comprises a suction assembly 110, an extension pipe assembly 130, a cleaner body 150 (hereinafter, canister body), and an antibiotic and sterilization material aerosol-generating unit 170.

The suction assembly 110 moves in contact with a cleaning surface to draw in dust or contaminant-laden air, and comprises a suction port 111 drawing in dust or contaminant-laden air at the bottom portion, and an air suction path 113 guiding the drawn air to the extension pipe assembly 130.

The extension pipe assembly 130 comprises an extension pipe 131 communicating with the air suction path 113 of the suction assembly 110, and a flexible hose 133 of which an end is connected with the extension pipe 131 and the other end is connected to an air inlet 151a of the canister body 150 to fluidly communicate with a dust collecting chamber 151.

Referring to FIG. 2, a dust-collecting chamber 151 and a motor-driving chamber 155 are formed by partitions 153a, 153b in the canister body 150. The dust-collecting chamber 151 employs a dust-collecting unit 180 such as a dust bag 181 to collect dusts or contaminants in air drawn from the suction assembly 110 (refers to FIG. 1). The partition 153a comprises an air path hole 153a' passing air cleaned from the dust collecting unit 180, and the air path hole 153a' comprises a filter member 183 refiltering contaminants in the cleaned air.

The motor driving chamber 155 comprises a motor assembly 190 generating a suction force. The partition 153b and the canister body 150 comprises air outlets 153b', 157 at each of rear sides to discharge air, which is cleaned through the dust collecting chamber 151 collecting dusts and contaminants and then passes through the motor driving chamber 155.

The aerosol-generating unit 170 sprays antibiotic and sterilization material of minute particles onto the air path in which air drawn from the air inlet 151a moves. The aerosol-generating unit 170 generates aerosols of nano sizes from antibiotic and sterilization materials such as powdery or plate-like Argentum or silver (Ag) material to spray them onto the air path. The aerosol-generating unit 170 may be provided in any place of the air path. However, the aerosol-generating unit 170 may be preferably provided at a side of the inlet 151a of the canister body 150 which connects the extension pipe assembly 130 and the dust collecting chamber 151 in the canister type vacuum cleaner with the changeable suction assembly 110 as shown in FIG. 2. The aerosol-generating unit 170 is not limited to be provided in the dust collecting chamber 151, and may be provided in the air suction path 113 of the suction assembly 110, the extension pipe 131 of the extension pipe assembly 130, the flexible hose 133, or the motor driving chamber 153.

In the above description, "air path" is defined as a passage of air which is drawn from the air port 111 of the suction assembly 110, passes the dust collecting chamber 151 and the motor driving chamber 153, and then flows out of the canister body 150. The silver nano aerosol may be generated by electric heating or electric discharge method. The aerosol-generating unit 170 is periodically operated while the motor assembly 190 is driven to perform cleaning so that the silver nano aerosol can be sprayed.

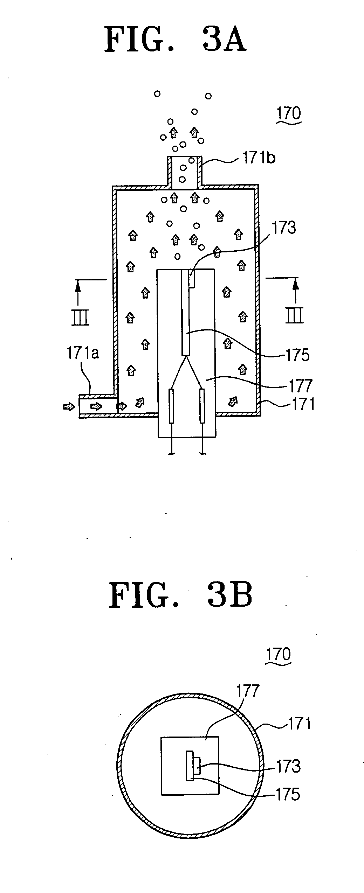

FIG. 3A is a sectional view of a small size of an aerosol-generating unit applied to an embodiment of the present invention, and FIG. 3B is a sectional view of the aerosol- generating unit taken on III-III line of FIG. 3A.

Referring to FIGS. 3A and 3B, the aerosol-generating unit 170 comprises an aerosol-generating unit body 171 forming an air path in which air flows, an aerosol material receiving part 173 formed in the body 171 to receive the nano aerosol generating material, a heater 175 capable of heating the nano aerosol material, and an insulator 177 blocking the leak of the heat generated from the heater 175. The aerosol-generating unit body 171 comprises an air inlet 171 a at an end for air to flow in, and an aerosol exhaust opening 171b at the other end for the generated nano particles to flow out with the flowing air.

By the above-mentioned structure, if the temperature of the heater 175 increases, the temperature of the material in the aerosol material receiving part 173 also increases so as to reach the vaporizable temperature. If the temperature is the vaporization temperature or more, vaporization occurs on a surface of the material and the vaporized material moves by relatively cold air around the material. At this time, the instant decrease of temperature changes vapor elements to solid particles of nano-meter sizes. The nano particles with air flow out of the aerosol exhaust opening 171b of the aerosol-generating unit body 171 to be sprayed. Preferably, a fan (not shown) is formed at the air inlet 171a side, or a pressure is differentiated so that air can flow through aerosol-generating unit 170. In the present embodiment, a suction pressure of the motor assembly 190 may be used. The nano aerosol can reach anywhere air contacts due to the nano aerosol being easily diffused.

In the above embodiment, the heater 175 takes on configuration of a conic rod, however, this should not be considered as limiting. The heater 175 may be applied to various configurations such as a plate or a rectangular rod.

In the vacuum cleaner, as the motor assembly 190 is driven, the air suction port 111 of the suction assembly 110 draws in dusts or contaminants-laden air. The drawn air passes the extension pipe assembly 130 to move to the dust collecting chamber 151. The dusts or contaminants in air are collected in the dust bag 181, which is the dust collecting unit 180. The air cleaned from the dust collecting unit 180 passes the air path hole 153a' and then the motor driving chamber 155. The air is discharged to the outside via the air exhaust openings 153b', 157.

As described above, the aerosol-generating unit 170 formed at the inlet 151a side of the aerosol-generating unit body 150 periodically sprays the Argentum or silver (Ag) nano aerosol. Accordingly, the sprayed aerosol is mixed with air which is drawn in and flown from the cleaning surface so as to function antibiotic and sterilization against dusts. The sprayed aerosol functions antibiotic and sterilization to dusts or contaminants in the dust collecting chamber 151 as well as the flowing air.

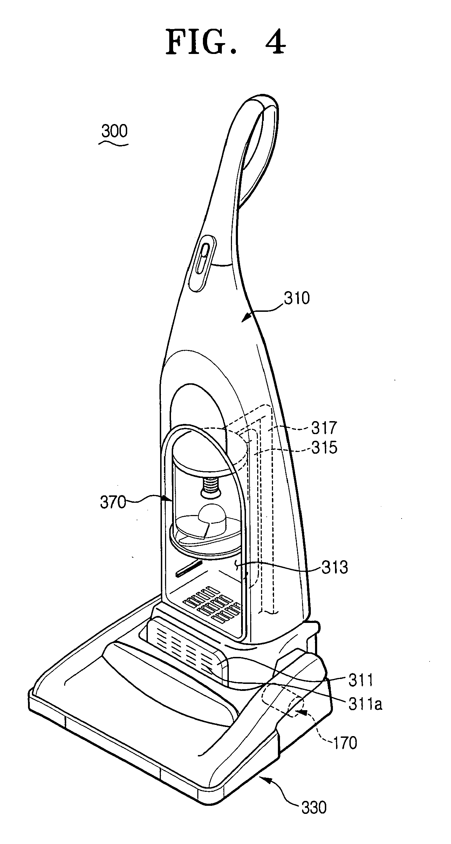

FIG. 4 is a view of an upright type vacuum cleaner according to another embodiment of the present invention.

Referring to FIG. 4, an upright type vacuum cleaner 300 comprises an upright cleaner body 310 (hereinafter, upright body), an upright cleaner suction assembly (hereinafter, upright suction assembly) 330, and an aerosol-generating unit 170.

The upright body 310 comprises a motor driving chamber 311 of the upright cleaner (hereinafter, upright driving chamber) and a dust collecting chamber 313 of the upright cleaner (hereinafter, upright dust collecting chamber) which are partitioned up and down. The upright suction assembly 330 is attached to a bottom portion of the upright body 310 to rotate by certain angles.

The upright motor driving chamber 311 comprises a motor assembly (not shown) generating a suction force, and an air discharge opening 311 a discharging drawn air.

The dust-collecting unit 313 comprises a dust-collecting unit such as a cyclone dust-collecting unit 370. The cyclone dust-collecting unit 370 is connected with each of an end of an air inflow path 315 and an end of an air discharge path 317 in the upright body 310. The other end of the air inflow path 315 is fluidly communicated with the upright suction assembly 330. The other end of the air discharge path 317 is connected with the upright motor driving chamber 311. Dusts and contaminants-laden air is drawn in via the upright suction assembly 330 from the cleaning surface. The drawn air passes the air inflow path 315 to flow in the cyclone dust-collecting unit 370, the dusts and contaminants are filtered via the cyclone dust-collecting unit 370, and then the filtered air flows via the air discharge path 317 and the upright motor driving chamber 311 so as to be discharged to the outside. The cyclone dust-collecting unit 370 is not an important part for the present invention, and therefore, detailed description thereof will be omitted for the sake of brevity.

The suction assembly 330 comprises an aerosol-generating unit 170 to function antibiotic and sterilization to the drawn air and dusts or contaminants in the drawn air. The detailed constructions and operations of the aerosol-generating unit 170 will be substituted with the above descriptions.

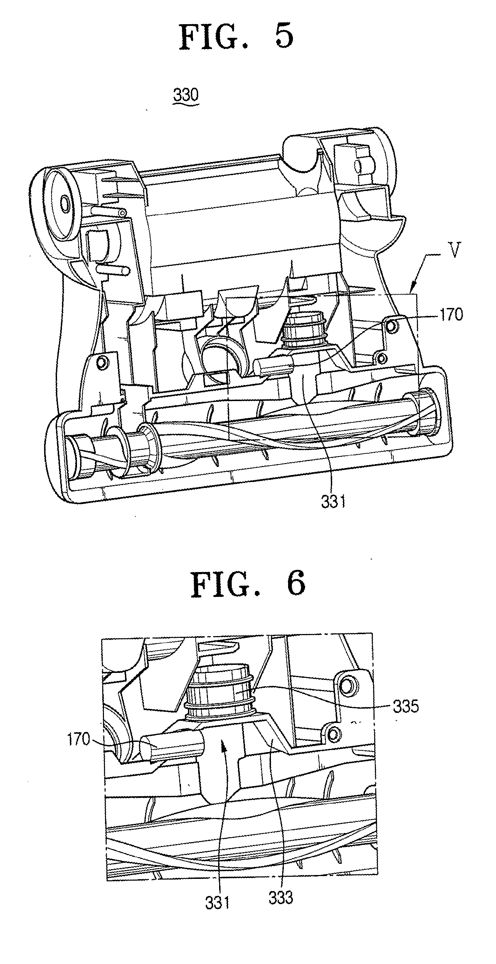

FIG. 5 is a view of the aerosol-generating unit 170 installed to the upright suction assembly 330 of the upright vacuum cleaner 300 according to an embodiment of the present invention, and FIG. 6 is an enlarged view of portion V of FIG. 5.

Referring to FIGS. 5, 6, the upright suction assembly 330 comprises at the bottom an air suction path 331 (hereinafter, upright air suction path) to draw in dusts or contaminants-laden air in contact with the cleaning surface. The air suction path 331 is formed by a guide rib 333. The air suction path 331 further comprises a connection pipe 335 connecting the air suction path 331 and the air inflow path 315 (refer to FIG. 4). The air inflow path 331 adjacent to the connection pipe 335 comprises the aerosol-generating unit 170 to function antibiotic and sterilization to the drawn air and dusts or contaminants in the air. The detailed description of the aerosol-generating unit 170 will be omitted since constructions thereof are the same as the above. The aerosol-generating unit 170 is provided in the upright air suction path 331 of the upright suction assembly 330 so that areas contacting the drawn dusts or contaminants can be maximized and the antibiotic and sterilization effect can be maximized. In the present embodiment, the aerosol-generating unit 170 is provided in the upright suction assembly 330. However, this should not be considered as limiting. The aerosol-generating unit 170 may be provided in anyplace of air path with flowing air therein such as the upright dust collecting chamber 313 and the upright motor driving chamber 311.

In the above embodiments, the present invention is applied to the canister type vacuum cleaner and the upright type vacuum cleaner by way of an example. However, this should not be considered as limiting, but the present invention may be applicable to various types of vacuum cleaners.

Additionally, the antibiotic and sterilization material is not limited to the Argentum or silver (Ag), but may be substituted with another material such as Aurum or gold (Au) with a high antibiotic and sterilization effect.

While the invention has been shown and described with reference to certain embodiments thereof, it will be understood by those skilled in the art that various changes in form and details may be made therein without departing from the spirit and scope of the invention as defined by the appended claims.

## Claims

1. A vacuum cleaner comprising:
a body with a dust-collecting chamber and a motor driving chamber;
a suction assembly with an air suction path;
a dust collecting unit formed in the dust-collecting chamber and collecting dust or contaminants from an air;
a motor assembly formed in the motor driving chamber and generating an air suction force; and
an aerosol-generating unit provided in an air path where the air drawn from the suction assembly flows, and spraying an antibiotic and sterilization material as a minute particle size.

2. The vacuum cleaner according to claim 1, wherein the aerosol-generating unit is formed in the air suction path.

3. The vacuum cleaner according to claim 1, wherein the aerosol-generating unit is formed in the dust-collecting chamber.

4. The vacuum cleaner according to claim 1, wherein the aerosol-generating unit is formed in the motor driving chamber.

5. The vacuum cleaner according to any of claims 1 to 4, wherein the antibiotic and sterilization material comprises Argentum (Ag).

6. The vacuum cleaner according to any claims 1 to 4, wherein the antibiotic and sterilization material comprises Aurum (Au).

7. The vacuum cleaner according to any of claims 1 to 6, wherein the aerosol-generating unit is periodically operated.

8. The vacuum cleaner according to any of claims 1 to 7, wherein the aerosol-generating unit comprises:
a body with an air inflow opening at an end and an aerosol exhaust opening at the other end;
an aerosol material receiving part formed in the body and receiving an aerosol-generating material;
a heater heating the aerosol material; and
an insulator blocking a heat generated from the heater.

9. A vacuum cleaner comprising:
a body with a dust collecting chamber and a motor driving chamber;
a suction assembly connected with the body and drawing in an air;
an extension pipe assembly connecting the suction assembly and the dust collecting chamber of the body;
a dust collecting unit formed in the dust-collecting chamber and collecting dusts or contaminants from the air;
a motor assembly formed in the motor driving chamber and generating an air suction force; and
an aerosol-generating unit formed at an inflow opening side of the body connected with the extension pipe assembly and spraying an antibiotic and sterilization material as a minute particle size.

10. The vacuum cleaner according to claim 9, wherein the antibiotic and sterilization material comprises Argentum (Ag).

11. The vacuum cleaner according to claim 9, wherein the antibiotic and sterilization material comprises Aurum (Au).

12. The vacuum cleaner according to any of claims 9 to 11, wherein the aerosol-generating unit is periodically operated.

13. The vacuum cleaner according to any of claims 9 to 12, wherein the aerosol-generating unit comprises:
an aerosol-generating unit body with an air inlet at an end and an aerosol exhaust opening at the other end;
an aerosol material receiving part provided in the body and receiving an aerosol generating material;
a heater heating the aerosol material; and
an insulator blocking a heat generated from the heater.

14. A vacuum cleaner comprising:
a body with a motor-driving chamber and a dust-collecting chamber provided up and down therein;
a suction assembly provided at a lower portion of the body and having an air suction path;
a motor assembly provided in the motor-driving chamber;
a dust-collecting unit provided in the dust-collecting chamber; and
an aerosol-generating unit provided in the air suction path of the suction assembly and spraying an antibiotic and sterilization material as a fine particle.

15. The vacuum cleaner according to claim 14, wherein the antibiotic and sterilization material comprises Argentum (Ag).

16. The vacuum cleaner according to claim 14, wherein the antibiotic and sterilization material comprises Aurum (Au).

17. The vacuum cleaner according to any of claims 14 to 16, wherein the aerosol-generating unit is periodically operated.

18. The vacuum cleaner according to any of claims 14 to 17, wherein the aerosol-generating unit comprises:
an aerosol unit body with an air inlet at an end and an aerosol exhaust opening at the other end;
an aerosol material receiving part provided in the body and receiving an aerosol generating material;
a heater heating the aerosol material; and
an insulator blocking a heat generated from the heater.
